# EUROPEAN PATENT SPECIFICATION

(11) **EP 0 739 871 B1**
(45) Date of publication and mention of the grant of the patent: **15.12.1999**
(21) Application number: 95302836.2
(22) Date of filing: 25.04.1995
(51) Int. Cl.: C07C 2/54

(54) **Recovery and recycle of HF-amine complex in HF alkylation**
Zurückgewinnung und Kreislaufführung des HF-Amincomplexes bei der Alkylierung in Gegenwart von HF
Récupération et recirculation du complexe HF-amine dans l'alkylation en présence de HF

(43) Date of publication of application: 30.10.1996
(73) Proprietor: UOP, Des Plaines, Illinois 60017-5017 (US)
(72) Inventor: Kocal, Joseph A., Gurnee, Illinois 60031 (US); Hammershaimb, Harold U., Western Springs, Illinois 60553 (US); Cornish, Robert J., Carpentersville, Illinois 60110 (US); Marker, Terry L., Warrenville, Illinois 60555 (US); Himes, James F., Mount Prospect, Illinois 60056 (US)
(74) Representative: Brock, Peter William

(56) References cited:
- US-A- 5 073 674

## Description

The field of the present invention is hydrocarbon processing. The invention relates to catalytic alkylation of hydrocarbons employing a liquid hydrogen fluoride (HF)-amine complex such as pyridine in a mixture.

### BACKGROUND

Alkylation using HF is a widely used commercial refining and petrochemical process. Generally, alkylation is the addition of an alkyl group to another hydrocarbon. Commercially, HF is used to alkylate isobutane with propylene, butylene, and amylene isomers to produce high octane gasoline blending components, as well as to alkylate benzene in the manufacture of detergents.

HF is known to be an extremely hazardous substance and chemical. HF has properties peculiar to itself which call for special handling and treatment. Health and environmental concerns over HF have led to a search for ways to minimize the possibility that an accidental release of HF would flash and form a hazardous HF cloud. One approach has been to complex HF with suitable compounds that reduce the vapor pressure of HF and the tendency of the HF to form an aerosol.

Certain nitrogen-containing compounds, such as pyridine, picolines, quinoline, trimethylamine, and triethylamine, are known to form complexes with HF and to reduce its volatility. See JP-A-57(1982)-92502. Olah in US-A-5073674 disclosed that mixtures of HF and preferred nitrogen-containing compounds (complexing agents) such as ammonia, methylamines, ethylamines, propylamines, butylamines, pentylamines, pyridine, picolines, melamine, and hexamethylene-tetramine remained effective catalysts in alkylation of alkanes by alkenes. Mixtures of Olah's preferred complexing agents with HF are hereinafter referred to as HF-amine complexes.

Such HF-amine mixtures are not suitable for economical use in prior art alkylation processes that use HF, however, because of the cost of adding fresh complexing agent as make-up. The prior art processes tend to concentrate the complexing agent in a stream that is rejected from the process. This happens because ASO, or acid-soluble oil, is soluble in the HF-amine complexes. ASO is a recognized term in the art of alkylation, and is sometimes referred to as HF-soluble oil, polymer, conjunct polymer, or polymer by-product. ASO is soluble in HF. In alkylation processes, ASO is an alkylation by-product that is generally formed by oligomerization of reactants or by reactions of impurities in the charge stock to the alkylation process. As such, ASO may have a variety of different compositions and physical properties depending on the reactants of the alkylation process, the operating conditions, etc. ASO is formed in both a motor fuel alkylation process and a detergent alkylation process. ASO may be in the boiling range of 149 to 482°C (300 to 900°F). It may also contain other non-hydrocarbon elements, including halogens, oxygen, nitrogen, sulfur, etc. As those skilled in the art of HF alkylation are aware, ASO generally decreases the activity of the HF catalyst. This effect can be beneficial at relatively low concentrations of ASO, but at higher concentrations it has an overall detrimental effect on the process. Therefore, in those processes where ASO is formed, ASO must be removed at least periodically from the circulating HF by a process that is commonly referred to as regeneration. In a typical HF-alkylation process, regeneration typically includes distilling or stripping HF from a stream of HF and ASO, returning the HF to the alkylation process, and rejecting the ASO. But, because HF-amine complexes typically have boiling points within the boiling range of ASO, complexing agent exits the process with the ASO and consequently the amount of complexing agent in the process is depleted. This ultimately results either in an increase in the volatility of the circulating HF stream, which may increase the potential for flash atomization, or else a requirement to add fresh complexing agent into the process as make-up, which may be uneconomical. Thus, there is a need for a method of separating the complexing agent from the ASO, so the separated complexing agent can be efficiently and economically recycled within the process.

US-A-3959402 discloses a method of separating a stream containing ASO, dissolved HF, and CBM or "constant boiling mixture." CBM is a recognized term in the art of alkylation and refers to an azeotropic mixture of HF and water. The method comprises first stripping the stream to remove at least some of the HF, and then separating the HF-depleted stream into a CBM-rich phase and an ASO-rich phase.

US-A-5191150 discloses a method of separating a stream containing ASO, HF, and sulfolane by first separating out HF and then gravitationally separating the HF-depleted stream into a polymer-rich stream and a sulfolane-rich stream.

US-A-5237122 discloses a method of separating a liquid containing HF and a sulfone compound by first adding water and then settling out an ASO phase and a sulfone with water phase.

### BRIEF SUMMARY

The present invention is a process for recovering HF-amine complex from a by-product stream containing ASO, HF, and HF-amine complex. The process first removes at least some of the HF from the feed stream to form an HF-lean stream, and then separates the HF-lean stream into a stream enriched in ASO and another stream enriched in the HF-amine complex. This process is a particularly effective method of removing ASO from a mixture in which a Lewis base such as Olah's preferred complexing agents is used to reduce the tendency of HF to form an aerosol.

The present invention can be distinguished from prior art processes by the surprisingly different properties that a mixture of HF and amine complexing agent has in comparison to a mixture of HF and water. The manner in which HF associates with pyridine and the like complexing agent and the properties that result from that association are unique. Whereas pyridine for example is quite unlike water in that it is soluble in ASO, it has now been discovered that certain mixtures of HF and pyridine are surprisingly like the azeotropic mixture of HF and water in that they are almost immiscible in ASO. The present invention takes advantage of this surprising discovery about mixtures of HF and amine complexing agents such as pyridine to separate HF-amine complexes from ASO in a particularly effective and economical process. In processes that use HF and from which ASO is rejected at least intermittently, this invention may be used to separate, recover, and recycle the amine complexing agent before the ASO is disposed of. Therefore, this invention makes economical the use of pyridine and the like complexing agents to reduce the volatility of HF in processes that form ASO.

Accordingly this invention provides a process for removing an ASO from a stream containing ASO, HF and HF-amine complex. In a separation zone, at least a portion of the HF is selectively removed from the stream to produce an HF-lean stream having an HF to amine complexing agent mole ratio of 3:1 to 5:1. In another separation zone, the HF-lean stream is separated into a stream enriched in ASO and a stream enriched in HF-amine complex.

In a second embodiment, this invention is a method of removing ASO from a feed stream containing ASO, HF, and an HF-pyridine complex, the ASO having been formed in an alkylation process catalyzed by a mixture containing HF and pyridine. In a stripping zone, at least a portion of the HF is selectively removed from the feed stream to produce an HF-lean stream having an HF to pyridine mole ratio of 3:1 to 5:1. In a phase separation zone, the HF-lean stream is separated into a stream enriched in ASO and a stream enriched in HF-pyridine complex, with the latter returned to a reaction zone of the alkylation process.

The Figure is a simplified flow diagram of an embodiment of the invention.

The feed stream charged to the subject process contains an amine complexing agent such as pyridine, HF and ASO. The mixture may also contain water. The feed stream may be produced by an HF alkylation process that is catalyzed by a mixture containing HF-amine complex.

Lewis bases that can function as complexing agents contain any one of the Group 5A elements, namely nitrogen, phosphorus, arsenic, antimony, and bismuth. The HF-Lewis base complex is in a form which is sufficiently polar that it is not very soluble in ASO, which is generally non-polar, and so the HF-Lewis base and ASO are separable by phase separation. The more polar the HF-Lewis base complex, the more facile is the separation between the HF-Lewis base complex and the ASO. And, within the group consisting of the Group 5A elements, the corresponding Lewis base generally becomes more polar as the molecular weight of the element decreases, because the electron cloud around the nucleus of the element becomes more localized and less diffuse as the molecular weight decreases. And so the likelihood that the HF-Lewis base complex will be sufficiently polar that the phase separation will be facile increases as the molecular weight of the Group 5A element decreases. For this reason, the Group 5A element which is used according to the invention is that with the lowest molecular weight, namely nitrogen. The complexing agents containing nitrogen can be: one of the chemical formula NR₁R₂R₃, where R₁, R₂, and R₃ can be alkyl, aryl or hydrogen, including ammonia, methylamine, ethylamine, propylamines, butylamines, pentylamines, dimethylamine, trimethylamine, diethylamine, triethylamine, diphenylamine, dibenzylamine, and aniline and alkyl- and aryl-substituted anilines including N,N-dimethylaniline; pyridine, and alkyl- and aryl-substituted pyridines, especially methyl-substituted pyridines, including picolines, lutidines, and collidine; a polycyclic compound, such as quinoline; a compound having more than one nitrogen atom, including noncyclic compounds such as ethylenediamine and cyclic compounds such as imidazoles; and a cyclic compound having other kinds of atoms such as oxygen in the ring, including morpholine. The preferred complexing agent is pyridine. The feed stream may contain more than one complexing agent.

In a mixture with HF, the Lewis base may complex with HF to varying degrees. The tendency of Lewis bases to form complexes with HF is described in JP-A-57(1982)-92502, as well as in US-A-5073674. Although the extent of complexing depends on the type of Lewis base and its amount in a mixture relative to the amount of HF, the exact degree to which the Lewis base complexes with HF is uncertain. JP-A-57-92502 teaches that under certain conditions one pyridine molecule complexes with twenty HF molecules. As a working hypothesis, it is believed that the HF molecules that are in close proximity to the Lewis base are more tightly complexed (e.g., by ionic inter-molecular forces), while HF molecules that are further from the Lewis base are more loosely complexed (e.g., by Van der Waals forces). JP-A-57-92502 also teaches that under certain conditions one pyridine molecule forms a stable complex with three HF molecules. A mixture comprising HF and a Lewis base may be comprised of several HF-Lewis base complexes, each having a different number of HF molecules for each Lewis base molecule. Thus, it is to be understood that the term "HF-Lewis base complex" refers to one or more HF-Lewis base complexes. As a working hypothesis, it is believed that the extent to which the Lewis base complexes with HF generally decreases as the molecular weight of the Group 5A element increases. Thus, HF will tend to complex more extensively with the nitrogen-containing Lewis bases used according to the invention than with bismuth-containing Lewis bases. In any event, to the extent that the Lewis base does complex with HF, the ease of phase separation of the Lewis base from the ASO may depend on the actual HF-Lewis base complex that is present in the mixture, rather than on the Lewis base itself. In the present invention, the concentration of complexing agent in the feed stream is generally between 1 and 50 wt-% on an ASO-free basis. The concentration of HF in the feed stream is generally between 50 and 99 wt-% on an ASO-free basis. This typically corresponds to an HF to complexing agent mole ratio substantially in excess of 5:1.

Within these broad ranges of Lewis base and HF concentrations lie various narrower ranges for particular Lewis bases. For any particular Lewis base, its concentration in a feed stream from an HF alkylation process will depend on its physical properties and on its effect in vapor pressure suppression and in alkylation. For example, where the Lewis base is pyridine, the concentration of pyridine in the feed stream is generally from 5 wt-% to 25 wt-% on an ASO-free basis, and preferably from 10 wt-% to 20 wt-% on an ASO-free basis. Accordingly, for the pyridine case the concentration of HF in the feed stream is generally from 75 wt-% to 95 wt-% on an ASO-free basis, and preferably from 180 wt-% to 190 wt-% on an ASO-free basis.

This invention is directed towards a stream containing an amine complexing agent, HF and ASO, with the most preferred amine complexing agent being pyridine. The following explanation is focused on the use of pyridine as a preferred embodiment of the present invention.

The particular composition of the ASO is not essential to the operation of this invention, and this invention is useful with any suitable ASO as described above. The operating conditions of this invention which are suitable for separation of ASO from a mixture containing ASO, HF and pyridine will depend on the ASO. Without undue experimentation, one skilled in the art can determine operating conditions that are suitable for different ASO compositions. It is not intended to limit the scope of this invention to any particular ASO composition. The concentration of ASO in the feed stream is generally between 0.5 and 25 wt-%, but this is not a critical element of the invention.

Water may be present in the feed stream. In an HF alkylation process, water may enter the process with the feed hydrocarbons and then dissolve in the HF, ultimately being rejected as an azetropic mixture of HF and H₂O. As the concentration of water in the HF increases, an HF-water mixture becomes more corrosive. In order to prevent water accumulating in the alkylation process and causing corrosion, water is typically separated from the circulating HF. The concentration of water in the feed stream is generally between 0.1 and 3.0 wt-% on an ASO-free basis.

In accordance with this invention, first at least a portion of the HF in the feed is removed. The particular method of removing the HF from the feed is not an essential feature of this invention. The method may also have the effect of removing from the feed some pyridine, perhaps complexed with HF, from the feed. Generally, however, the method employed will remove HF in a greater proportion than pyridine. Although the method of removal may be flashing, distillation or extraction, the preferred method of removing HF from the feed mixture is by stripping. Such a scheme is shown in US-A-3249650, in which a stream containing isobutane and other light hydrocarbons is introduced into the bottom of a stripping column, which those skilled in the art of alkylation commonly refer to as a regenerator or rerun column. Although the regenerator may comprise a flash chamber or a packed bed column, it is preferably a trayed column. The heat for the separation may be provided by a stab-in type or a kettle type reboiler in the regenerator, however the heat is preferably provided to the stripper via the stripping medium, which can provide at least most and preferably all of the heat input into the stripper. Alternatively, the feed may be preheated. The stripping medium may be nitrogen, hydrogen, methane, ethane, propane, or any other non-reactive gas. For a motor fuel alkylation process, the stripping medium may contain pentane, but preferably it contains isobutane which is heated or superheated. For a detergent alkylation process, the stripping medium may contain benzene, but preferably no stripping medium is used. It is an essential feature that this HF separation step be operated to produce an HF-depleted stream containing an HF to complexing agent mole ratio of 3:1 to 5:1.

In the bottoms product of the stripping column, which is referred to herein as the HF-lean or HF-depleted stream, the concentration of HF is generally between 20 and 80 wt-% on an ASO-free basis. Where the Lewis base is pyridine, the concentration of HF in the HF-lean stream is generally between 40 wt-% and 70 wt-% on an ASO-free basis, and preferably between 43 and 65 wt-% on an ASO-free basis. Within these ranges, the lower the concentration of HF, the more facile is the subsequent separation of the HF-pyridine complex from the ASO, but the higher are the capital and operating costs of the stripping.

The process conditions under which HF is removed from the feed stream include temperatures in the range of from 10 to 260°C (50 to 500°F), with 52 to 232°C (125 to 450°F) being preferred, and pressures in the range of from 138 to 1724 kPa (20 to 250 psi), with 586 to 1379 kPa (85 to 200 psi) being preferred.

The stripping column bottoms product, or HF-lean or HF-depleted stream, is passed into a zone for separating the ASO from the HF-pyridine complex. The preferred method of separation is gravity-settling. Gravity-settling can be accomplished in a variety of mechanical devices, but the simplest device employs a quiescent zone that allows the HF-lean stream to separate by gravity difference into an ASO-enriched hydrocarbon phase and a HF-pyridine-complex-enriched acid phase. Accordingly, two process streams, an ASO-enriched hydrocarbon stream and an HF-pyridine-complex-enriched acid stream, are recovered from the separation device. In an alkylation process, the ASO-enriched hydrocarbon stream is generally rejected from the process, and the HF-pyridine-complex-enriched acid stream is recycled to the reaction zone of the process.

In more general terms, the method of separating the ASO from the HF-amine complex depends on the particular amine complexing agent used and on the conditions of the first separation zone. Where the effluent stream is a liquid and the complexing agent is also a liquid, the method of separation would typically be gravity-settling as described above, but it may also comprise flashing, distillation, centrifugation, extraction, and any suitable method that separates on the basis of differences in density, miscibility, or both.

Generally, the concentration of complexing agent in the ASO-enriched hydrocarbon stream is between 0.1 and 5 wt-%, but preferably the concentration is between 0.1 and 1.0 wt-%. In an HF alkylation process employing pyridine, the lower the concentration of pyridine in the ASO-enriched stream, the lower is the quantity of pyridine that is lost from the process, because the ASO-enriched stream is generally rejected from the process. As also mentioned above, the consequences of losing pyridine from an alkylation process may be an increase in the volatility of the circulating HF which may increase the potential for flash atomization, or an increase in the rate at which pyridine is made up to the process, which is costly. In this context, it is important to note that another important advantage of the present invention is that the concentration of pyridine in the ASO-enriched stream is relatively low.

Generally, the concentration of ASO in the HF-amine-complex-enriched acid stream is between 0.1 and 50 wt-%. Where the amine is pyridine, the concentration of ASO in the HF-amine-complex-enriched stream is generally between 0.1 wt-% and 25 wt-%, and preferably the concentration is between 0.1 and 15 wt-%. In an HF alkylation process employing pyridine, the lower the concentration of ASO in the HF-pyridine-complex-enriched stream, the lower the amount of ASO that is recycled to the alkylation process. Reducing the concentration of ASO that is recycled to an HF alkylation process is desirable because this reduces the size of the acid regenerator. Alternatively, for a given charge rate to a given size of acid regenerator, reducing the concentration of ASO that is recycled to an HF alkylation process is desirable because this reduces the concentration of ASO in the circulating HF, which allows higher complexing agent concentrations and greater flash suppression.

The process conditions under which the HF-lean stream is separated into an ASO-enriched hydrocarbon stream and an HF-amine-complex-enriched acid stream include temperatures in the range of from -18 to 260°C (0 to 500°F) with 149 to 260°C (300 to 500°F) being preferred when the ASO has been formed in a detergent alkylation process. A temperature of 66 to 149°C (150 to 300°F) is preferred when the ASO has been formed in a motor fuel alkylation process. Pressures in the range of from 138 to 1724 kPa (20 to 250 psi), preferably with the pressure being sufficient to minimize or prevent vaporization and to maintain the streams in a liquid phase.

The concentration of ASO in the HF-pyridine-complex-enriched acid stream can be further reduced by stripping the HF-pyridine-complex-enriched acid stream again to remove more HF, and then separating by phase separation into two streams. One advantage of further lowering of the concentration of HF is that even more ASO can be separated from the HF-pyridine-complex-enriched acid stream. This improved separation does not come without cost, because additional stripping and gravity-settling steps add both capital and operating costs to the process. Depending on the design of the stripping and settling zones, and the number of zones, the concentration of ASO in the HF-pyridine-complex-enriched acid stream that results from successive stripping and settling can be reduced to levels below 1.0 wt-%. The process conditions at which HF is further stripped from the HF-pyridine-complex-enriched acid stream include temperatures in the range of from about 10 to 260°C (50 to 500°F), with 38 to 232°C (100 to 450°F) being preferred, and pressures in the range of from 138 to 1724 kPa (20 to 250 psi), with 586 to 862 kPa (85 to 125 psi) being preferred. In HF-alkylation processes, ASO is removed from the process on average at a rate that is equivalent to its rate of formation. The optimum concentration of ASO in the HF-pyridine-complex-enriched acid stream that is returned to the alkylation process depends on technical and economic factors, including the capacity and operating costs of the regenerator and the desired concentration of ASO in the alkylation process. In general, for any given rate of ASO production and concentration of ASO in the alkylation process, the higher the concentration of ASO in the HF-pyridine-complex-enriched acid stream that is returned to the process, the greater must be the feed rate to the regenerator. This is because the ASO that is returned to the alkylation process with the HF-pyridine-complex-enriched acid stream must ultimately be rejected by the regenerator. Thus, this invention is particularly advantageous for alkylation processes wherein the target ASO concentration is relatively low and the feed rate capacity of the regenerator is limited.

Another embodiment of the subject invention is useful when water is present in the feed stream. In an HF alkylation process, high concentrations of water in the circulating acid are undesirable because of the potential for corrosion. Therefore, it is desirable to maintain the concentration of water at an acceptable level in the process. In the present invention, a substantial amount of water in the feed stream may be removed using the separation zone that removes a portion of the HF from the feed stream. As described above, this separation zone may be a stripping column, in which case the overhead stream would contain the HF removed from the feed stream, as well as some or most of the water in the feed stream. However, this separation zone may also be operated so that a significant portion of the water is not removed with the overhead stream. Thus, the stripping column bottoms product, or the HF-lean stream, could contain a substantial amount of the water that entered the process in the feed stream. The HF-lean stream passes into a separation zone, such as a gravity-settling zone, that produces an ASO-enriched hydrocarbon stream and a HF-pyridine-complex-enriched acid stream that comprises most of the water that entered with the HF-lean stream.

One embodiment of the present invention removes a substantial portion of the water in the HF-pyridine-complex-enriched stream. This is another surprising aspect of this invention, because one would have expected that the water in the HF-pyridine-complex-enriched stream would form a pyridine-water azeotrope or a tertiary azeotrope of pyridine-HF-water. If either of these azeotropes had formed, then removing water from the process would have had the undesirable consequence of removing the pyridine, too. Instead, the formation of the pyridine-water azeotrope or the pyridine-HF-water azeotrope is not observed. The explanation for this is that the pyridine is sufficiently complexed with HF that it is unavailable to form an azeotrope with water.

Taking advantage of this phenomenon, a simple and effective method has been found to reduce the water concentration in the HF-pyridine-complex-enriched acid stream before it returns to the alkylation process. The HF-pyridine-complex-enriched acid stream may be passed at least intermittently into a stripping column to remove water. This column may be a reboiled distillation column, or it may be a column that employs a stripping stream such as isobutane to strip the water from the HF-pyridine-complex-enriched acid stream. The heat required for the separation may be provided by a reboiler, or some or all of it may be provided by the stream used for stripping. Alternatively, some of the heat for the separation may be provided by preheating the HF-pyridine-complex-enriched acid stream before it enters the stripping column. Although one would have expected such a column to produce either an overhead stream containing pyridine and significant water in the form of a pyridine-water azeotrope or a bottoms stream containing significant water in the form of a pyridine-HF-water azeotrope, the stripping column produces an overhead stream that contains HF and water but very little pyridine and a bottoms stream that contains significant pyridine but very little water. The water concentration of the overhead stream is generally from 5 wt-% to 15 wt-% and is preferably 10 wt-%.

The process conditions under which water is removed from the HF-pyridine-complex-enriched acid stream include temperatures in the range of from 10 to 260°C (50 to 500°F), with 38 to 232°C (100 to 450°F) being preferred. Pressures should be in the range of from 138 to 1742 kPa (20 to 250 psi), with 586 to 852 kPa (85 to 125 psi) being preferred.

The bottoms stream of this H₂O stripping column is generally returned to the alkylation process, although if the bottoms stream contains significant ASO it may be sent to phase separation to remove ASO and then the ASO-depleted bottoms stream would be returned to the alkylation process. The overhead stream may be rejected at least intermittently from the process and sent directly to neutralization. Alternatively the overhead stream can be sent to an additional separation zone which removes HF overhead for return to the alkylation process and produces a bottoms product comprising CBM (azeotropic mixture of HF and H₂O) that is rejected from the process and sent to neutralization.

In this last embodiment of the invention, the advantage of an additional separation zone that produces a bottoms product comprising CBM is that the loss of HF to neutralization is significantly reduced. This additional separation zone may be a stripping column to remove HF. This column may be a reboiled distillation column, or it may be a column that employs a stripping stream such as isobutane. The function of this stripping column is to strip HF from the overhead stream that contains HF and water. The heat required for the separation may be provided by a reboiler, or some or all of it may be provided by the stream used for stripping. Alternatively, the heat for separation may be provided by preheating the overhead stream before it enters the column. Thus, this reduced loss of HF does not come without cost, because the additional stripping step adds both capital and operating costs to the process. But depending on the frequency of water removal, the amount of water removed, and the costs of neutralization and byproduct disposal, the use of a stripping column may be economically justifiable. Where an additional stripping zone is used to strip HF, the concentration of water on a hydrocarbon-free basis in the HF stream that is returned to the alkylation process is between 0.1 and 5 wt-% water, and preferably it is between 0.1 and 1 wt-% water.

The process conditions under which HF is removed from the HF/H₂O overhead stream include temperatures in the range of from 38 to 260°C (100 to 500°F), with 49 to 232°C (120 to 450°F) being preferred. Pressures should be in the range of from 138 to 1724 kPa (20 to 250 psi), with 586 to 862 kPa (85 to 125 psi) being preferred.

Referring now to a preferred embodiment shown in the Figure, a mixture from an alkylation process, containing HF, an HF-pyridine complex, ASO, and water is charged in line 10 into a first stripper 12. In the first stripper 12, HF is selectively removed from the feed stream in an overhead stream through lines 14 and 36, and returns to the alkylation process through a line 38. The bottoms stream of the first stripper is depleted in HF and is passed through a line 16 into a second stripper 18. In the second stripper 18, HF is selectively removed from the bottoms stream of the first stripper under conditions selected to result in a mole ratio of HF to complexing agent of 3:1 to 5:1. The bottoms stream of the second stripper is passed through a line 22 into a gravity settler 24. In the gravity settler, the bottoms stream of the second stripper separates by phase separation into a settler overhead stream enriched in ASO and a settler bottoms stream enriched in the complex. The settler overhead stream is passed through a line 26 to facilities that neutralize and dispose of the ASO. The settler bottoms stream is passed through a line 28 and returns to the alkylation process through the line 38. The overhead stream of the second stripper which contains both HF and water passes through a line 20 into a third stripper 30. In the third stripper 30, water is selectively removed from the overhead stream of the second stripper. The bottoms stream of the third stripper 30 is enriched in water and is passed through a line 32 to facilities that neutralize the HF and dispose of the water. The overhead stream of the third stripper 30 is enriched in HF, passes through a line 34 and the line 36, and returns to the alkylation process through the line 38.

### Example 1

This example shows the high degree of separation of ASO and HF-pyridine complex that can be achieved as a result of reducing the HF content of a mixture containing HF, pyridine, and ASO. A mixture containing HF-pyridine complex and ASO was subjected to HF stripping and then separated into a first ASO-rich phase and a first complex-rich phase. The first complex-rich phase contained 50.3 wt-% HF, had an HF-to-pyridine molar ratio of 5 and contained about 9.7 to 10.5 wt.% ASO. The first ASO-rich phase contained 0.12 to 0.5 wt-% pyridine (after neutralization) and 3.2 to 3.3 wt-% HF. Then, the first complex-rich phase was subjected to HF stripping again and separated into a second ASO-rich phase and a second complex-rich phase. The second complex-rich phase contained 42.9 wt-% HF, had an HF-to-pyridine molar ratio of 3, and contained less than 1.0 wt.% ASO. The second ASO-rich phase contained about 0.3 wt-% pyridine (after neutralization) and about 0.5 wt-% HF.

Thus, an alkylation catalyst containing HF, ASO and pyridine can be separated into a pyridine-rich stream containing less than about 1.0 wt-% ASO which can be returned to the alkylation process and an ASO-rich stream containing between 0.12-0.5 wt-% pyridine that can be rejected from the process.

### Example 2

This example shows that a pyridine-water azeotrope does not tend to form in an HF-pyridine-water mixture. A mixture of 140 g HF, 36 g water, and 79 g pyridine was distilled at atmospheric pressure. Two distinct temperatures were observed at which a constant boiling overhead temperature was maintained. The first was at 112° C, which corresponds to the boiling point of CBM (azeotropic mixture of HF and H₂O) and the second was at 182°C, which corresponds to the boiling point of the HF-pyridine complex that has an HF-to-pyridine molar ratio of 3.

### Example 3

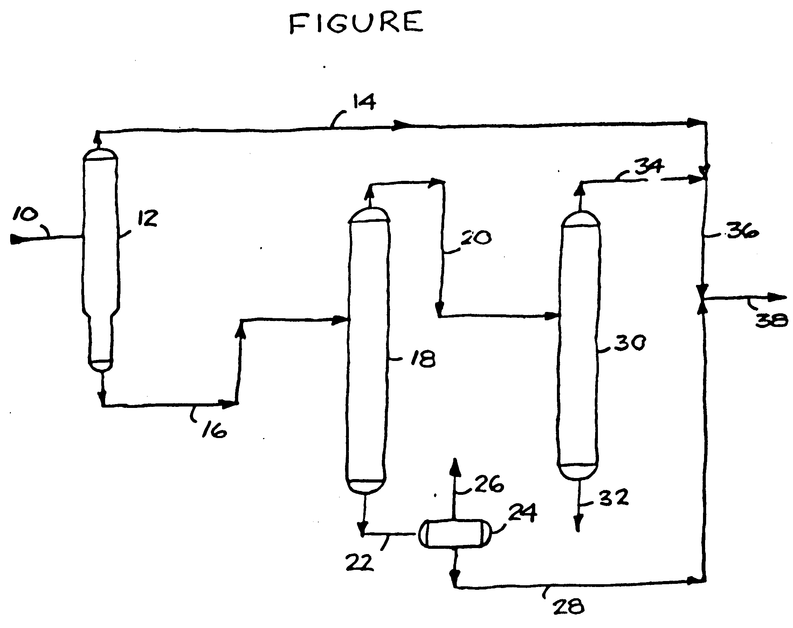

Table 1 shows an example of the composition of some of the streams of the process shown in Figure 1. This example is based on conventional engineering calculations and laboratory experiments. In the gravity settler, the second stripper bottoms stream is effectively separated into an overhead stream that contains 99% ASO and is disposed of and a bottoms stream that contains less than 1% ASO and returns to the alkylation process.

**TABLE 1**

| Stream: | Feed Stream 10 | First Stripper Bottoms 16 | Second Stripper Bottoms 22 | Settler Overhead 26 | Settler Bottoms 28 | Second Stripper Overhead 20 | Third Stripper Overhead 34 | Third Stripper Bottoms 32 |
|---|---|---|---|---|---|---|---|---|
| | | | | | | | | |

| Composition in wt-%: | | | | | | | | |
|---|---|---|---|---|---|---|---|---|
| HF | 79 | 55 | 40 | <1 | 43 | 85 | 94 | 38 |
| Pyridine | 15 | 36 | 52 | 1 | 56 | 1 | <1 | 4 |
| ASO | 4 | 6 | 7 | 99 | <1 | 4 | 5 | 2 |
| Water | 2 | 3 | 1 | <1 | 1 | 10 | 1 | 57 |
| Total | 100 | 100 | 100 | 100 | 100 | 100 | 100 | 100 |

## Claims

1. A hydrocarbon alkylation process using an HF-amine complex catalyst having a molar ratio of HF to complexing agent above 5:1 wherein an acid soluble oil (ASO)-by-product-containing-stream [10] is formed containing unacceptable amounts of such complex, characterized in that a portion of said complex is recovered for recycle to the alkylation step by the steps of:
(a) selectively removing a portion of the HF from the by-product stream [10] to produce an HF-depleted stream [22] containing an HF to complexing agent mole ratio of 3:1 to 5:1;
(b) separating the resulting HF-depleted stream [22] into a hydrocarbon phase [26] enriched in ASO and an acid phase [28] depleted in ASO and containing a substantial portion of the complex; and
(c) recycling the acid phase [28] to the hydrocarbon alkylation step.

2. The process of Claim 1 further characterized in that the HF-depleted stream [22] contains HF at a concentration of not more than 80 wt% on an ASO-free basis.

3. The process of Claim 1 or 2 further characterized in that the hydrocarbon phase [26] contains the complexing agent at a concentration between 0.1 and 5 wt% on an ASO-free basis.

4. The process of Claim 2 or 3 further characterized in that step (a) operates at a temperature of from 10 to 260°C (50 to 500°F) and at a pressure of from 138 to 1724 kPa (20 to 250 psi).

5. The process of Claim 1 further characterized in that step (b) operates at a temperature of from -18 to 260°C (0 to 500°F) and a pressure of from 138 to 1724 kPa (20 to 250 psi).

6. The process of any of Claims 1 to 5 wherein step (a) is performed in a stripping zone in which HF is selectively removed from the by-product stream [10] by contacting such stream with isobutane to produce the HF-depleted stream [22].

7. The process of any one of Claims 1 to 6 further characterized in that the by-product stream [10] contains H₂O which is removed therefrom in step (a) with the HF stream [20] and subsequently separated at least in part from the HF overhead stream [20] by stripping in an H₂O separation column [30].

8. The process of any one of Claims 1 to 7 wherein the complexing agent is pyridine.

## Patentansprüche

1. Kohlenwasserstoffalkylierungsverfahren unter Verwendung eines HF-Amin-Komplex-Katalysators mit einem HF/Komplexbildner-Molverhältnis über 5:1, bei dem ein säurelösliches Öl (ASO) als Nebenprodukt enthaltender Strom [10] mit unvertretbar hohen Gehalten an derartigem Komplex anfällt, dadurch gekennzeichnet, daß man den Komplex teilweise zur Rückführung in den Alkylierungsschritt zurückgewinnt, indem man:
(a) aus dem Nebenproduktstrom [10] das HF teilweise selektiv entfernt, wobei man einen HF-abgereicherten Strom [22] mit einem HF/Komplexbildner-Molverhältnis von 3:1 bis 5:1 erhält;
(b) den erhaltenen HF-abgereicherten Strom [22] in eine mit ASO angereicherte Kohlenwasserstoffphase [26] und eine ASO-abgereicherte und einen wesentlichen Teil des Komplexes enthaltende Säurephase [28] trennt und
(c) die Säurephase [28] in den Kohlenwasserstoffalkylierungsschritt zurückführt.

2. Verfahren nach Anspruch 1, ferner dadurch gekennzeichnet, daß der HF-abgereicherte Strom [22] HF in einer Konzentration von höchstens 80 Gew.-%, bezogen auf ASO-freie Basis, enthält.

3. Verfahren nach Anspruch 1 oder 2, ferner dadurch gekennzeichnet, daß die Kohlenwasserstoffphase [26] den Komplexbildner in einer Konzentration zwischen 0,1 und 5 Gew.-%, bezogen auf ASO-freie Basis, enthält.

4. Verfahren nach Anspruch 2 oder 3, ferner dadurch gekennzeichnet, daß man Schritt (a) bei einer Temperatur von 10 bis 260°C (50 bis 500°F) und einem Druck von 138 bis 1724 kPa (20 bis 250 psi) durchführt.

5. Verfahren nach Anspruch 1, ferner dadurch gekennzeichnet, daß man Schritt (b) bei einer Temperatur von -18 bis 260°C (0 bis 500°F) und einem Druck von 138 bis 1724 kPa (20 bis 250 psi) durchführt.

6. Verfahren nach einem der Ansprüche 1 bis 5, bei dem man Schritt (a) in einer Strippzone durchführt, in der HF durch Inberührungbringen des Nebenproduktstroms [10] mit Isobutan selektiv aus dem Strom entfernt wird, wobei man den HF-abgereicherten Strom [22] erhält.

7. Verfahren nach einem der Ansprüche 1 bis 6, ferner dadurch gekennzeichnet, daß der Nebenproduktstrom [10] H₂O enthält, welches in Schritt (a) mit dem HF-Strom [20] daraus entfernt und anschließend durch Strippen in einer H₂O-Trennsäule [30] zumindest teilweise aus dem HF-Überkopfstrom [20] abgetrennt wird.

8. Verfahren nach einem der Ansprüche 1 bis 7, bei dem man als Komplexbildner Pyridin einsetzt.

## Revendications

1. Procédé d'alkylation d'hydrocarbure utilisant un catalyseur complexe HF-amine ayant un rapport molaire du HF à l'agent complexant supérieur à 5:1, dans lequel est formé un courant contenant comme sous-produit une huile soluble en milieu acide (ASO) [10] contenant des quantités inacceptables de ce complexe, caractérisé en ce qu'une partie dudit complexe est récupérée pour un recyclage vers l'étape d'alkylation par les étapes consistant à :
(a) enlever sélectivement une partie du HF du courant de sous-produit [10] pour produire un courant appauvri en HF [22] ayant un rapport molaire du HF à l'agent complexant de 3:1 à 5:1 ;
(b) séparer le courant appauvri en HF [22] résultant en une phase d'hydrocarbure [26] enrichie en ASO et une phase acide [28] appauvrie en ASO et contenant une grande partie du complexe ; et
(c) recycler la phase acide [28] vers l'étape d'alkylation d'hydrocarbure.

2. Procédé selon la revendication 1, caractérisé de plus en ce que le courant appauvri en HF [22] contient HF à une concentration ne dépassant pas 80 % en poids calculée en excluant l'ASO.

3. Procédé selon la revendication 1 ou 2, caractérisé de plus en ce que la phase d'hydrocarbure [26] contient l'agent complexant à une concentration comprise entre 0,1 et 5 % en poids en excluant l'ASO.

4. Procédé selon de la revendication 2 ou 3, caractérisé de plus en ce que l'étape (a) est conduite à une température de 10 à 260°C (50 à 500°F) et à une pression de 138 à 1724 kPa (20 à 250 psi).

5. Procédé de la revendication 1, caractérisé de plus en ce que l'étape (b) est exécutée à une température de -18 à 260°C (0 à 500°F) et à une pression de 138 à 1724 kPa (20 à 250 psi).

6. Procédé selon l'une quelconque des revendications 1 à 5, dans lequel l'étape (a) est conduite dans une zone d'extraction dans laquelle HF est retiré sélectivement du courant de sous-produit [10] par mise en contact de ce courant avec de l'isobutane pour produire le courant appauvri en HF [22].

7. Procédé selon l'une quelconque des revendications 1 à 6, caractérisé de plus en ce que le courant de sous-produit [10] contient de l'eau qui en est retirée dans l'étape (a) avec le courant de HF [20] et est ensuite séparée au moins en partie du courant de tête contenant HF [20] par extraction dans une colonne de séparation de H₂O [30].

8. Procédé de l'une quelconque des revendications 1 à 7, dans lequel l'agent complexant est la pyridine.
